# EUROPEAN PATENT APPLICATION

(11) **EP 1 609 428 A1**
(43) Date of publication of application: **28.12.2005**
(21) Application number: 05013498.0
(22) Date of filing: 22.06.2005
(51) Int. Cl.: A61B 17/32

(54) **Ultrasonic apparatus with selectable treatment modes**

(30) Priority: 22.06.2004 JP 2004184152
(71) Applicant: Olympus Corporation, Tokyo 151-0072 (JP)
(72) Inventor: Kimura, Kenichi, Hachioji-shi Tokyo 192-0014 (JP); Masuda, Shinya, Hino-shi Tokyo 191-0041 (JP); Shimizu, Koh, Hachiouji-shi Tokyo 192-0032 (JP)
(74) Representative: von Hellfeld, Axel

(57) **Abstract**

An ultrasonic surgical apparatus comprises a treatment device with an ultrasonic transducer driven to generate ultrasonic vibration, an operation device outputting a signal commanding the transducer to start driving in response to an operator's operation, and a driver driving the transducer by supplying current thereto. The apparatus further comprises a controller controlling the driver. The controller controls the driver using selectively a first output control pattern and a second output control pattern. The first output control pattern is set to supply the current of a first current value under control of constant current control in response to the signal from the operation device, while the second output control pattern is set to supply the current of a second current value in response to the signal from the operation device and then the current is reduced in amount as the time counted from starting the supply elapses.

## Description

### BACKGROUND OF THE INVENTION

### [Technical Field of the Invention]

This invention relates to an ultrasonic surgical apparatus for effecting treatment by ultrasonic vibration.

### [Related Art]

Ultrasonic coagulation and incision apparatuses, one type of apparatuses utilizing ultrasonic vibration and constituting surgical devices, have been used. The ultrasonic coagulation and incision apparatuses comprise an ultrasonic probe and a holder which is driven for opening and closing of the ultrasonic probe. Coagulation and incision of living tissues have thus been carried out by giving ultrasonic vibration to the ultrasonic probe in the state where the living tissues are held between the ultrasonic probe and the holder.

In such a conventional ultrasonic coagulation and incision apparatus, current control for maintaining the amplitude of an ultrasonic probe at a constant level, has been performed when ultrasonic waves are emitted.

The ultrasonic coagulation and incision apparatuses have been used in two ways of manipulations. One is to effect treatment to living tissues with comparatively high ultrasonic output, in which incision is carried out immediately after coagulation of the living tissues. Therefore, before proceeding to incision, there has been no ample time and thus it has been difficult to visually confirm as to whether or not the coagulation satisfactorily took place.

The other is to effect treatment to living tissues with comparatively low ultrasonic output of a level which would not incise the living tissues. In this manipulation, coagulation of living tissues can be visually confirmed, however, a problem has been that more time was required for the coagulation due to the low ultrasonic output.

### SUMMARY OF THE INVENTION

The present invention has been made in view of the circumstances described above, and has as its object to provide an ultrasonic surgical apparatus which reliably enables coagulation of living tissues in a short time.

According to the present invention, as one aspect, there is provided an ultrasonic surgical apparatus comprising: a treatment device with an ultrasonic transducer driven to generate ultrasonic vibration in response to supply of current; an operation device outputting a signal commanding the ultrasonic transducer to start driving in response to an operator's operation; a driver driving the ultrasonic transducer by supplying the current thereto; and a controller controlling the driver to supply the current so that the current of a predetermined amount is supplied in response to the signal outputted from the operation device and then the current is reduced in amount as a time counted from starting the supply elapses.

Preferably, in the ultrasonic surgical apparatus, the controller is configured to control the driver based on an output control pattern with which the current of the predetermined amount is supplied in response to the signal outputted from the operation device and then the current is reduced in amount as the time counted from starting the supply elapses.

Still preferably, in the ultrasonic surgical apparatus according to the preferable configuration, the controller is configured to control the driver using selectively a first output control pattern and a second output control pattern, the first output control pattern being set to supply the current of a first current value under control of constant current control in response to the signal from the operation device and the second output control pattern being set to supply the current of a second current value in response to the signal outputted from the operation device and then the current is reduced in amount as the time counted from starting the supply elapses.

### BRIEF DESCRIPTION OF THE DRAWINGS

In the accompanying drawings:
Fig. 1 is a block diagram showing the configuration of an ultrasonic surgical apparatus according to a first embodiment of the present invention;
Fig. 2 is an enlarged view of a treatment portion of the device;
Fig. 3 is a block diagram showing the configuration of a control drive unit;
Fig. 4 is a graph explaining a first output control pattern in a transducer drive circuit;
Fig. 5 is a graph explaining a second output control pattern in a transducer drive circuit;
Fig. 6 is a graph explaining a modification of the second output control pattern in a transducer drive circuit;
Fig. 7 is a graph showing temperature changes of living tissues at the time of effecting ultrasonic treatment with respect to the output control patterns shown in Figs. 4 and 5;
Fig. 8 is a graph explaining a modification of an output control pattern;
Fig. 9 is a graph explaining another modification of an output control pattern;
Fig. 10 is a graph explaining still another modification of an output control pattern;
Fig. 11 is a graph explaining still another modification of an output control pattern;
Fig. 12 is a block diagram showing the configuration of a control drive unit according to a second embodiment of the present invention;
Fig. 13 is a rough flowchart explaining selection of control modes (output control patterns) implemented in the first embodiment;
Figs. 14A and 14B are illustrations explaining coagulation and incision, respectively, of living tissues; and
Fig. 15 is a rough flowchart explaining "being enable" and "being disenable" for ultrasonic driving based on monitoring of impedance, which is implemented in the second embodiment.

### DETAILED DESCRIPTION OF THE PREFERRED EMBODIMENTS

Various embodiments of the ultrasonic surgical apparatus according to the present invention are described hereinafter with referenced to the accompanying drawings.

### (First Embodiment)

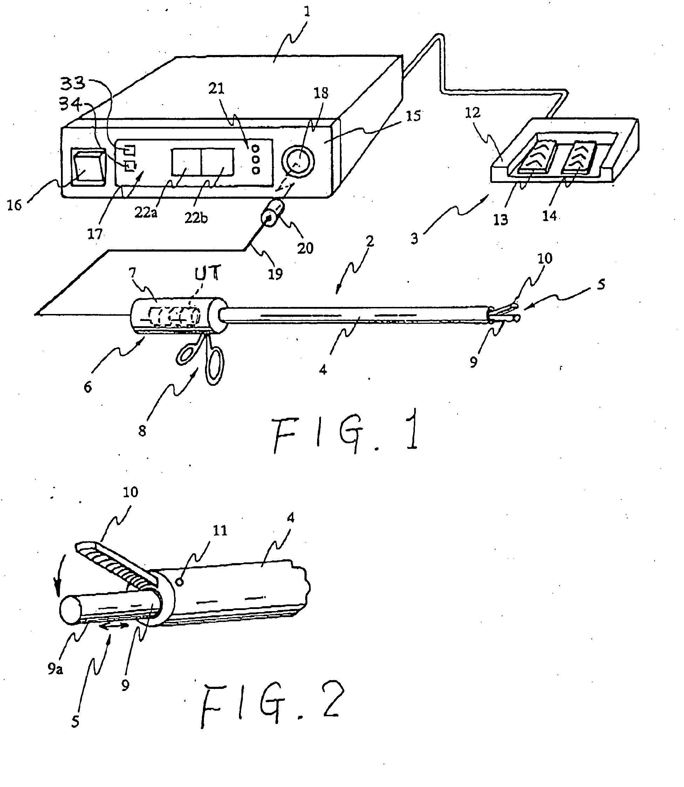

The ultrasonic surgical apparatus according to a first embodiment is described with reference to Figs. 1 to 7.

As shown in Fig. 1, the ultrasonic surgical apparatus according to the first embodiment comprises a control drive unit 1 as a device body, a hand piece 2, and a foot switch (FSW) 3 as switching means. The hand piece 2 and the foot switch 3 are physically and electrically connected to the control drive unit 1.

The hand piece 2 is provided with a treatment portion 5 which is disposed at the tip of an elongated and cylindrical sheath 4, and with a handling portion 6 which is disposed at the base of the sheath 4. It should be noted that, in the present embodiment, the end of the sheath 4, which is located near the control drive unit 1 is referred to as a base or base portion (or base side), and the opposite end of the sheath 4 is referred to as a tip or a tip portion (or tip side).

The handling portion 6 comprises a case 7 for accommodating an ultrasonic transducer UT which generates ultrasonic vibration, and a handle 8 disposed at the case 7. An operator can operate the treatment portion 5 by operating the handle 8.

The sheath 4 is provided therein with a probe 9 for transferring ultrasonic vibration from the ultrasonic transducer UT to the treatment portion 5. The tip portion of the probe 9 is exposed outside from the tip of the sheath 4.

As shown in Fig. 2, the probe 9 is provided with a holder 10 which is driven for opening and closing of an exposed tip portion 9a of the probe 9. The holder 10 is linked to the tip of the sheath 4 so as to be rotatable about a rotating pin 11. Upon operation of the handle 8 by an operator, the holder 10 is driven for opening and closing of the exposed tip portion 9a. As a result, living tissues can be held between the probe 9 and the holder 10.

The foot switch 3 comprises a switch body 12, and two operation pedals 13 and 14 set up on a floor or the like, which are independently operated from the switch body 12.

A control panel 15 is provided at the front face of the control drive unit 1. The control panel 15 is provided with a power switch 16, an operation display panel 17, and a connecting portion 18. One end of a cable 19 is linked to the handling portion 6 of the hand piece 2. A connector 20, which is disposed at the other end of the cable 19, is detachably connected to the connecting portion 18 mentioned above.

The operation display panel 17 is provided with a setting device 21 for setting a pattern for output control (hereinafter referred to "output control pattern") of ultrasonic waves when effecting ultrasonic treatment; a first display 22a for indicating a first output control pattern set at the setting device 21; and a second display 22b for indicating a second output control pattern set at the setting device 21.

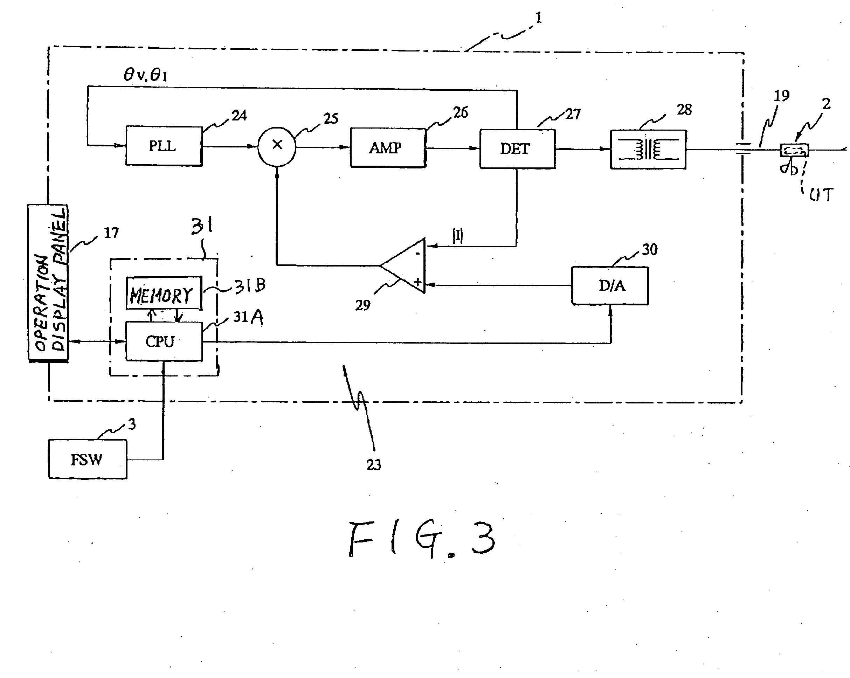

As shown in Fig. 3, an transducer drive circuit 23 is incorporated into the control drive unit 1. In the present embodiment, the drive circuit 23 is configured to operate by employing a PLL control system as a resonance tracking system, and employing a current control system as an amplitude control system.

Particularly, the transducer drive circuit 23 comprises an output circuit having a phase-locked loop (PLL) circuit 24, a voltage control amplifier (VCA) 25 serving as an adder, a power amplifier (AMP) 26 which produces current for imparting power to an ultrasonic transducer, voltage/current detector (DET) 27 and an output transformer 28, which are connected in series in this order. The hand piece 2 is connected to an output port of the output transformer 28 through the connecting cable 19. The transducer drive circuit 23 is separated from the hand piece 2 in a fashion that a direct current is not passed therebetween.

The phase-locked loop circuit 24 is a circuit which tracks the resonant frequency of an ultrasonic transducer and drives it at the resonance frequency. The voltage/current detector (DET) 27 is connected to the phase-locked loop circuit 24. Further, the voltage/current detector (DET) 27 includes a circuit for detecting phase signals of voltage and current for performing a PLL operation, or for detecting the magnitude of a current that flows through an ultrasonic transducer.

The transducer drive circuit 23 further comprises a differential amplifier 29, a D/A converter 30, and a computer 31 which at least has a CPU 31A and a memory 31B. The computer 31 is connected with the D/A converter 30, the foot switch (FSW) 3, and the operation display panel 17. The voltage/current detector (DET) 27 is connected to one input terminal of the differential amplifier 29, and the D/A converter 30 is connected to the other input terminal.

A program data effective as a soft ware procedure for controlling ultrasonic output is stored beforehand in the memory 31B. Thus, the CPU 31A reads the program data at the time of starting up from the memory 31B to execute the program. The execution also includes reading of the information on the operation that has been performed by an operator for the operation display panel 17 and the foot switch 3.

As a result, with the execution of such a control, the CPU 31A outputs a command current (digital signal) to the D/A converter 30, by which the magnitude of a current corresponding to the output control pattern that has been set through the setting device 21 and the foot switch 3 is indicated.

The D/A converter 30 converts the command current for driving into an analogue signal for output to a noninverting input terminal of the differential amplifier 29. A current absolute value | I |, which has been detected by the voltage/current detector (DET) 27, is being given to an inverting input terminal of the differential amplifier 29, as a reference input. Further, an output terminal of the differential amplifier 29 is connected to the voltage control amplifier (VCA) 25. Accordingly, the differential amplifier (VCA) 29 compares the command current outputted from the D/A converter 30 with the magnitude | I | of the current detected by the voltage/current detector (DET) 27, and supplies a differential output to the voltage control amplifier (VCA) 25, so that the values of the command current and the magnitude | I | of the current are equalized. Thus, the magnitude of the voltage added to the ultrasonic transducer is adjusted, so that the value of a current that passes through the ultrasonic transducer is controlled to be the same as the preset output control pattern.

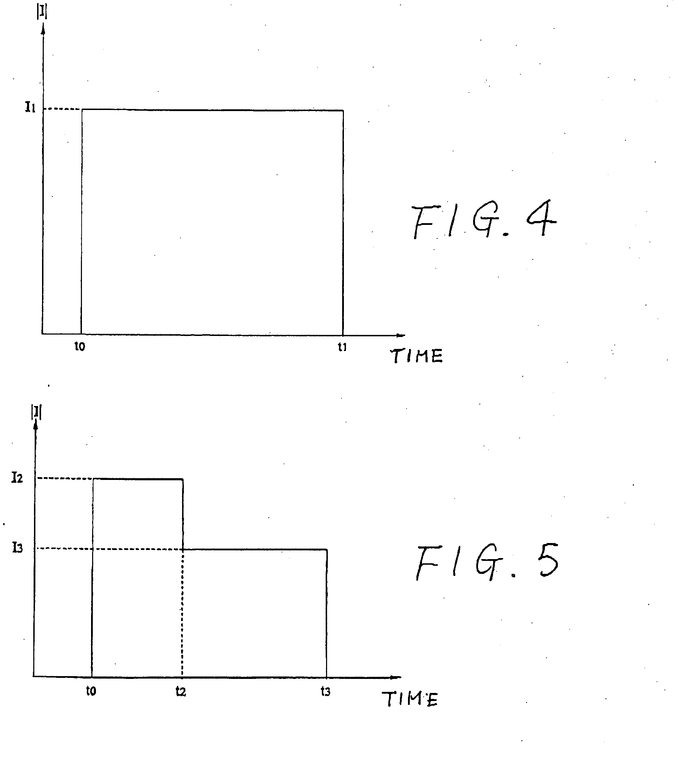

In the memory 31B of the computer 31, various kinds of output control patterns are stored in advance. The CPU 31A produces a command current for driving which is indicative of the magnitude of a current that passes through the ultrasonic transducer, based on the output control patterns which are stored in the memory 31B. Examples of such command currents are shown in Figs. 4 to 6, where the output control patterns are defined by the magnitude of the current | I | that passes through the ultrasonic transducer.

In the treatment portion 5, an amplitude of the ultrasonic vibration of the probe 9 is in proportion to the current | I | that passes through the ultrasonic transducer. Further, the magnitude of ultrasonic energy which effects in the coagulation and incision of living tissues is closely correlated with to the amplitude of the ultrasonic vibration of the probe 9 in the treatment portion 5. In other words, definition of the magnitude of the current | I | that flows through the ultrasonic transducer also defines the magnitude of the ultrasonic energy that effects in the coagulation and incision of living tissues.

The output control patterns shown in Figs. 4 to 6 are described hereunder.

Fig. 4 shows an output control pattern in which a constant first current I1 is allowed to pass through an ultrasonic transducer (i.e. constant current control) during an ON-operation (from time t0 to t1) of the operation pedal 13 or the operation pedal 14 of the foot switch 3.

Fig. 5 shows a output control pattern in which: on an ON-operation (time t0) of the operation pedal 13 or the operation pedal 14 of the foot switch 3, a second current I2 is allowed to flow; after lapse of a preset time t2, a third current I3, which is smaller than the second current I2, is allowed to flow; and when a preset time t3 has been reached, current is allowed to be zero (the ultrasonic output is switched OFF). In short, this control pattern shows output conditions where the current I is reduced stepwise.

In the output control pattern shown in Fig. 5, the second current I2 is set, as an example, at a value which is slightly larger (e.g., I2=I1×1.2) than the first current I1, and the third current I3 is set at a smaller value (e.g., I3=I1×0.6) than the first current I1 (see Fig. 4). Further, the time from t0 to t2 is set for approximately four seconds, and the time from t2 to t3 is set for approximately six seconds. In the control drive unit 1, display means 33 and sonic means 34 are provided, which notify at the time t3 that the ultrasonic output has been switched OFF (see Fig. 1).

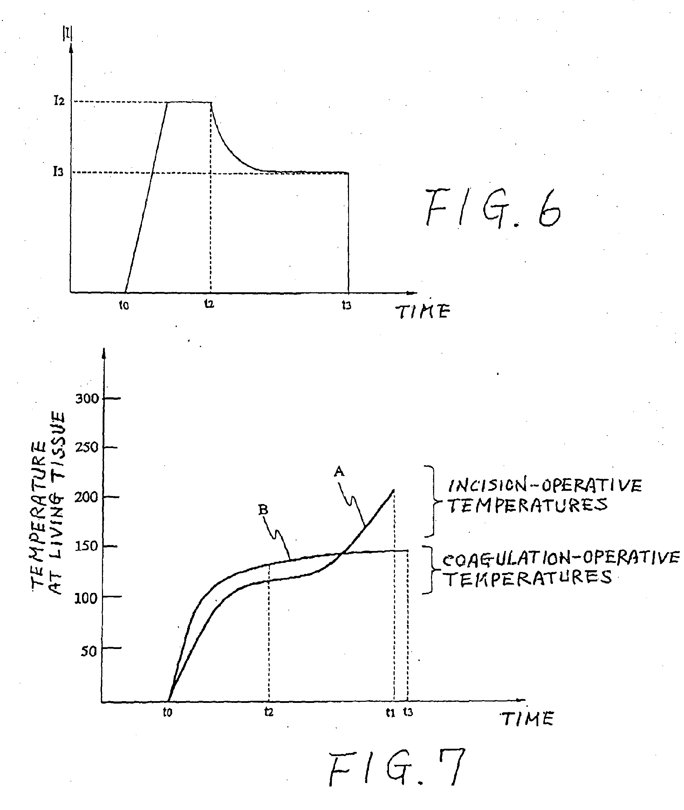

The output control pattern shown in Fig. 6 is a modification of the one shown in Fig. 5. The output control pattern shown in Fig. 6 provides conditions in which the current: increasingly becomes larger after the ON-operation (time t0) of the operation pedal 13 or the operation pedal 14 of the foot switch 3; reaches the value of the second current I2; is then gradually (continuingly) reduced, after lapse of the preset time t2, from the value of the second current I2 to the value of the third current I3; and is controlled so that the current value becomes zero (the ultrasonic output is switched OFF).

In the present embodiment, the currents have been described as satisfying a relation I3<U1<I2, however, the relation should not necessarily be limited to this relation. Further, the times t2 and t3 may be set at optimized values which mach the magnitudes of the currents I2 and I3, respectively.

The CPU 31A of the computer 31 is also in charge of display processing as follows. Specifically, in the setting device 21 of the operation display panel 17, on selection of a first output control pattern (e.g., the output control pattern shown in Fig. 4) as a first setting value, which has been stored in the memory 31B, letters or symbols (e.g., "COAG/CUT : 100%") for representing the selected first output control pattern, are displayed at the first display 22a. In the setting device 21, when a second output control pattern (e.g., the output control pattern shown in Fig. 5) is selected as a second setting value, the CPU 31A displays at the second display 22b, letters or symbols (e.g., "COAG : 100%") representing the selected second output control pattern.

The CPU 31A is set such that, upon ON-operation of the first operation pedal 13 of the foot switch 3 by an operator, it detects this ON-operation to drive the ultrasonic transducer UT with the first output control pattern, and that, on the other hand, upon ON-operation of the second operation pedal 14 of the foot switch 3, it drives the ultrasonic transducer UT with the second output control pattern, along its soft ware processing.

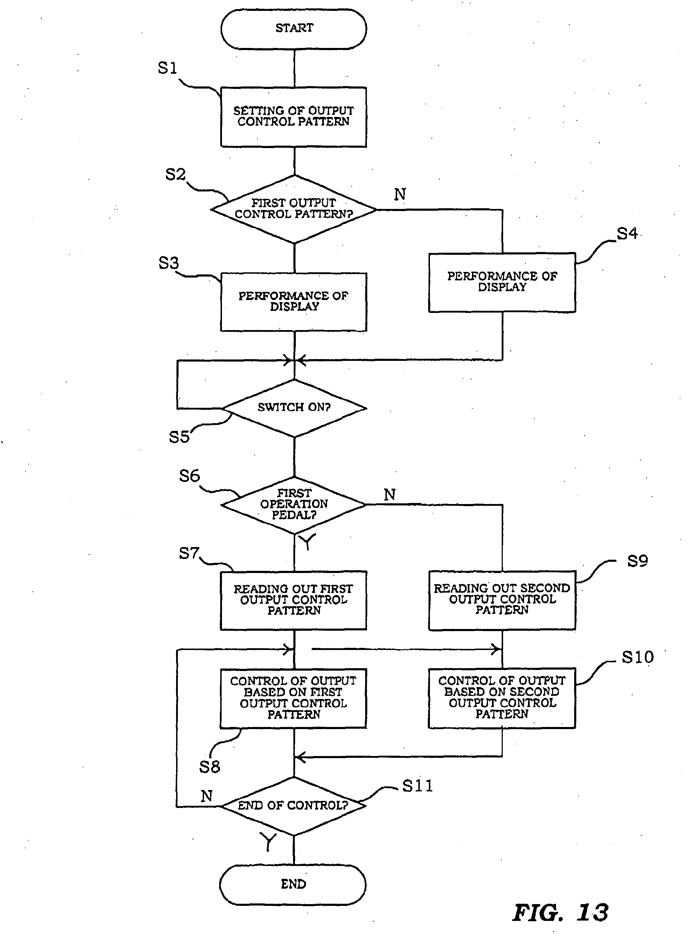

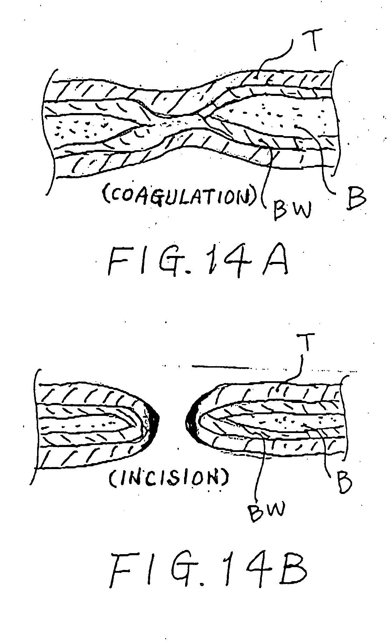

The effects of the ultrasonic surgical apparatus according to the present embodiment are described hereunder with reference to Figs. 13 and 14, in the context of the operation of ultrasonic treatment.

A power switch 16 of the control drive unit 1 is firstly switched ON to set an output control pattern (output conditions) (step S1 of Fig. 13). The setting of this output control pattern is performed by operating the setting device 21 of the operation display panel 17.

When an operator selects the first output control pattern (e.g., the output control pattern shown in Fig. 4) as the first set value through the setting device 21, letters or symbols for representing the selected first output control pattern are displayed (steps S2 and S3) at the first display 22a. Further, when the operator selects the second output control pattern (e.g., the output control pattern shown in Fig. 5) as the second set value through the setting device 21, letters or symbols representing the selected second output control pattern are displayed (steps S2 and S4) at the display 22b.

Then, the operator can have the living tissues be placed between the probe 9 and the holder 10 of the hand piece 2. In this state, the handle 8 of the handling portion 6 is operated in a closing direction to hold the tissues between the probe 9 and the holder 10.

After holding of the tissues, the operator can perform an ON-operation through the first operation pedal 13 of the foot switch 3, so that the ultrasonic transducer UT is driven (a first control mode: steps S5 to S8) with the first output control pattern. On the other hand, with an ON-operation of the second operation pedal 14 of the foot switch 3, the ultrasonic transducer UT is driven (a second control mode: Steps S5, S6, S9 and S10) with the second output control pattern.

Thus, for the first control mode (i.e., in the case of Fig. 4), the probe 9 undergoes ultrasonic vibration, so that the living tissues held between the probe 9 and the holder 10 are coagulated by the frictional heat generated by the ultrasonic vibration, and then are incised. Meanwhile, for the second control mode (i.e., in the case of Fig. 5), the probe 9 undergoes ultrasonic vibration, so that the living tissues held between the probe 9 and the holder 10 are firmly coagulated by the frictional heat generated by the ultrasonic vibration. In case of this second control mode, the processing of the living tissues is finished with the coagulation by an appropriate energy control. The reasons for this are described hereunder.

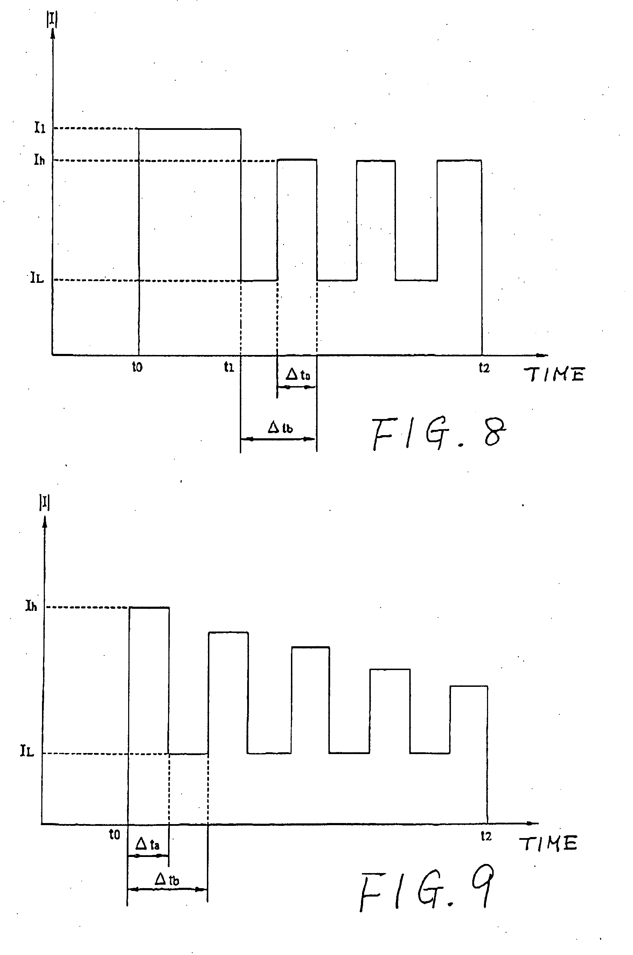

Fig. 7 shows temperature changes of living tissues at the time of the ultrasonic treatment. A line A shows the temperature changes when the treatment is effected with the first output control pattern (the output control pattern along the current waveform shown in Fig. 4). A line B shows the temperature changes when the treatment is effected with the second output control pattern (the output control pattern along the current waveform shown in Fig. 5).

In case of the first output control pattern, the temperature of the living tissues increases with the commencement (time t0) of the ultrasonic output triggered by the ON-operation of the first operation pedal 13 of the foot switch 3. Thereafter, the temperature increase declines once at about 100 to 150 degrees centigrade, where the electric power supplied to the ultrasonic transducer is consumed as energy for evaporating moisture contained in the living tissues. In the meantime, the living tissues are sufficiently coagulated. The state of coagulation is illustratively shown in Fig. 14A. In the figure, indicated by a reference T are tissues, by a reference B is a blood vessel (blood flow), and by a reference BW is a blood vessel wall.

Upon completion of the evaporation of moisture contained in the living tissues, the temperature of the living tissues begins increasing again. When the temperature has reached an incision-operative temperature (, for example, of about 200 degrees centigrade), incision of the living tissues is commenced. After the incision, the ultrasonic output is switched OFF with an OFF-operation (time t1) of the first operation pedal 13. In this way, incision is carried out in the state where the living tissues are coagulated, with the first output control pattern. The processes from the coagulation to the incision are shown in Figs. 14A and 14B. Fig. 14B shows a state where living tissues have been incised.

In case of the second control pattern, the temperature of the living tissues increases with the commencement (time t0) of the ultrasonic output triggered by the ON-operation of the second operation pedal 14 of the foot switch 3, where the current I2 of the second output control pattern is slightly larger than the current I1 of the first output control pattern. For this reason, the temperature of the living tissues changes slightly faster than in the case of the first output control pattern. The temperature increase is also declined at about 100 to 150 degrees centigrade.

In this case, as in the case of the first output control pattern, moisture contained in the living tissues are evaporated. After lapse of time t2, the current flowing through the ultrasonic transducer is switched to I3. Since the current I3 is sufficiently smaller than the current 11 of the first output control pattern, the temperature of the living tissues continues slightly increasing.

When time t3 is reached, the ultrasonic output is switched OFF. The fact that the ultrasonic output has been switched OFF is notified through the display means 33 or the sonic means 34 of the control drive unit 1.

After lapse of time t3, the temperature of the living tissues never reaches the incision-operative temperature. As shown in Fig. 7, for the second output control pattern, the time maintained at a coagulation-operative temperature is longer than in the case of the first output control pattern. Accordingly, for the second output control pattern, the living tissues are more firmly coagulated in a short time before being incised.

When the output control pattern shown in Fig. 6 is selected as the second output control pattern, the living tissues are more firmly coagulated in a short time before being incised as in the case of the output control pattern shown in Fig. 5.

As described above, treatment of living tissues with the second output control pattern as shown in Fig. 5 or 6 enables firm coagulation in a short time.

Selective operation of the operation pedal 13 or 14 of the foot switch 3 enables treatment that matches the states of the living tissues to be treated using the single hand piece 2. For example, when living tissues including a blood vessel of a comparatively small diameter are treated, the operation pedal 13 is operated to perform coagulation and incision with the first output control pattern. Conversely, when living tissues including a blood vessel of a comparatively larger diameter are treated, the operation pedal 14 is operated to perform firm coagulation with the second output control pattern.

As described above, according to the present embodiment, reliable coagulation of living tissues can be performed in a short time. Further, treatment depending on the states and kinds of the living tissues to be treated, is enabled using a single hand piece. Thus, according to the present embodiment, time can be saved which would otherwise have been required in making plural kinds of hand pieces be ready and in changing these hand pieces depending on the kinds or the like of living tissues. This may contribute to reducing time for surgery.

### (Modifications)

Hereinafter, several modifications of the first embodiment described above are described with reference to Figs. 8 to 11.

These modifications are associated with other examples of output control patterns than the ones that may be given to the ultrasonic transducer UT by the computer 31.

These output control patterns are stored in advance in the memory 31B of the computer 31. Thus, the CPU 31A reads the pattern data from the memory 31B to output a command current for driving to the D/A converter 30, based on the ever-changing pattern data. In this way, current controls along the output control patterns shown in Figs. 8 to 11 can be executed.

While the command currents based on the output control patterns may be ensured to issue from the CPU 31A, a function generator in which such patterns are stored in advance may be provided separately from the CPU, so that the outputs from the function generator may be ensured to be directed to the D/A converter 30 or the differential amplifier 29.

Fig. 8 shows an output control pattern of a first modification. In case of this output control pattern, upon an ON-operation (time t0) of the operation pedal 13 or the operation pedal 14 of the foot switch 3, the constant current 11 is flowed. The pattern is ensured to be of a waveform for altering the current | I | into the form of pulses after lapse of preset time t1.

In this modification, high level of pulsed output is represented by Ih, and its low level by IL, and an output duty of the high level Ih is set as Δta/Δtb. It is arranged such that when preset time t2 has been reached, the current | I | is rendered to be zero (i.e. the ultrasonic output is switched OFF).

In the control drive unit 1, the display means 33 and the sonic means 34 are provided to switch OFF the ultrasonic output when time t2 has been reached, as in the first embodiment.

In the present embodiment, although Il>Ih and IL>O, they may be Il=Ih and IL=0, respectively.

Fig. 9 shows an output control pattern of a second modification. In the output control pattern shown in Fig. 9, upon the ON-operation (time t0) of the operation pedal 13 or the operation pedal 14 of the foot switch 3, the current | I | is altered into the form of pulses from the very beginning. In this modification, a high level of pulsed output is represented by Ih, and its low level by IL, and an output duty of the high level Ih is set as Δta/Δtb. Unlike the previous modification, it is arranged such that the low level IL and the duty Δta/Δtb are fixed at constant values and that the high level Ih gradually reduces with the lapse of output time. Further, when preset time t2 is has been reached, the current | I | is ensured to be zero (i.e. the ultrasonic output is switched OFF).

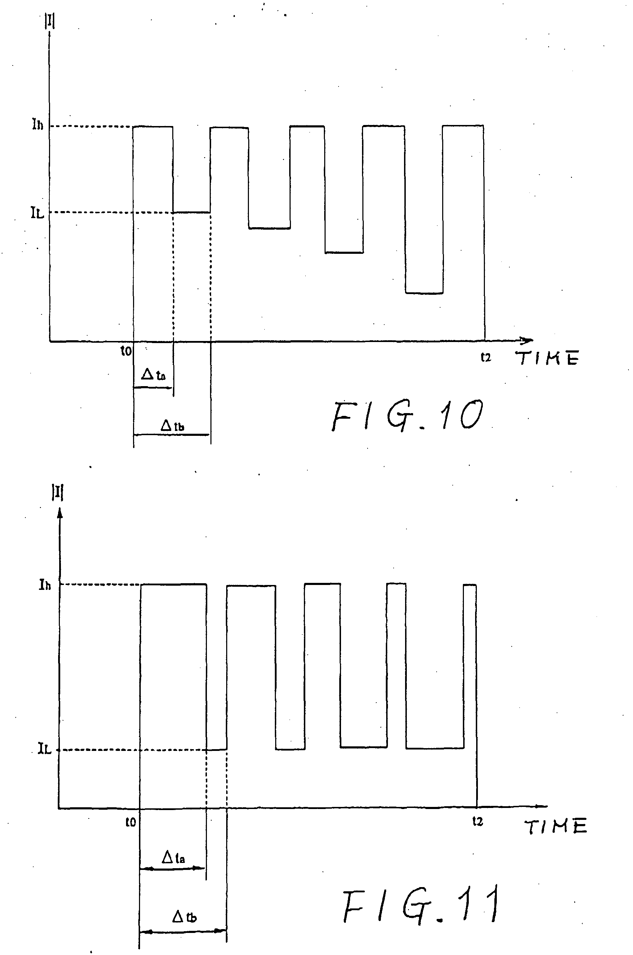

Fig. 10 shows an output control pattern of a third modification. In the output control pattern shown in Fig. 10, the high level Ih and the duty Δta/Δtb are fixed at constant values, and the low level IL is set to be gradually reduced with the lapse of output time.

Fig. 11 shows an output control pattern of a fourth modification. In the output control pattern shown in Fig. 11, the high level Ih and the low level IL are fixed at constant values, and the duty Δta/Δtb is set to be gradually reduced with the lapse of output time.

Modifications other than those shown in Figs. 9 to 11 may be provided, in which set values of each of the parameters Ih, IL and Δta/Δtb of output control patterns may be altered. For example, the high level Ih may be set at a constant value, and the low level IL and the duty Δta/Δtb may be set to be gradually reduced with the lapse of output time.

In any of the output control patterns shown in Figs. 8 to 11, the magnitude of the current | I | that flows through the ultrasonic transducer per unit time, i.e. the ultrasonic energy which effects in the coagulation and incision of living tissues, reduces with the lapse of output time. Accordingly, by giving ultrasonic treatment with the output control patterns shown in Figs. 8 to 11, firm coagulation can be ensured in a short time before incising the living tissues, as in the case of the output control patterns shown in Figs. 5 and 6 related to the first embodiment. As a result, the effects similar to those in the first embodiment may be obtained in these modifications.

### (Second Embodiment)

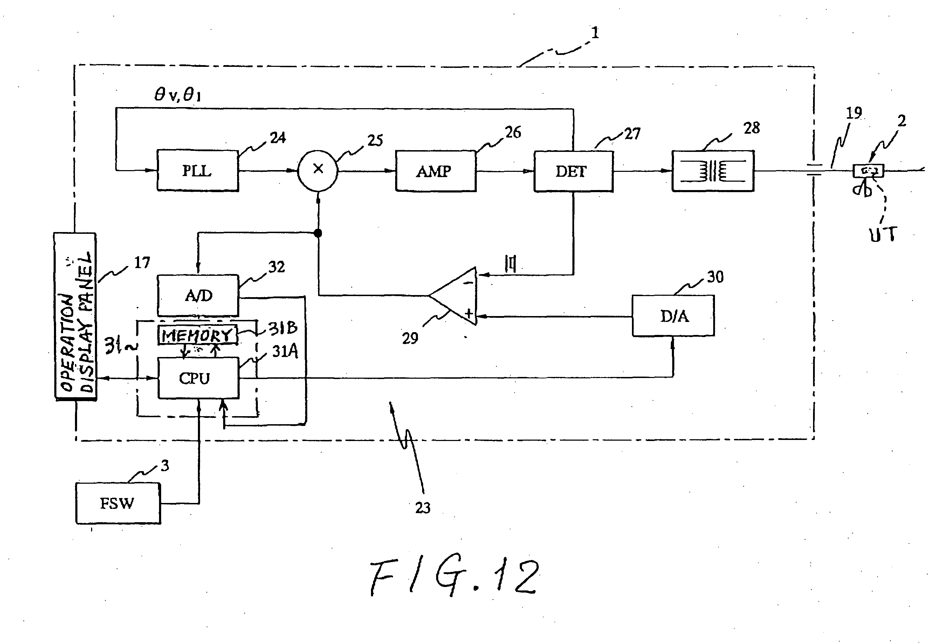

A second embodiment of the ultrasonic surgical apparatus is described hereinafter with reference to Fig. 12.

In the ultrasonic surgical apparatus of the present embodiment, like or the same components as those of the first embodiment are referred to by the same reference numbers, and description therefore is omitted or simplified.

Fig. 12 shows the transducer drive circuit 23 which is loaded on the control drive unit 1 of the ultrasonic surgical apparatus.

As shown, the transducer drive circuit 23 is provided with an A/D converter 32. The A/D converter 32 is connected between an output terminal of the differential amplifier 29 and the computer 31, so that the A/ D converter 32 converts output signals from the differential amplifier 29 into digital data and imparts the resultant to the computer 31.

The other portions of the configuration are the same with those described in the first embodiment.

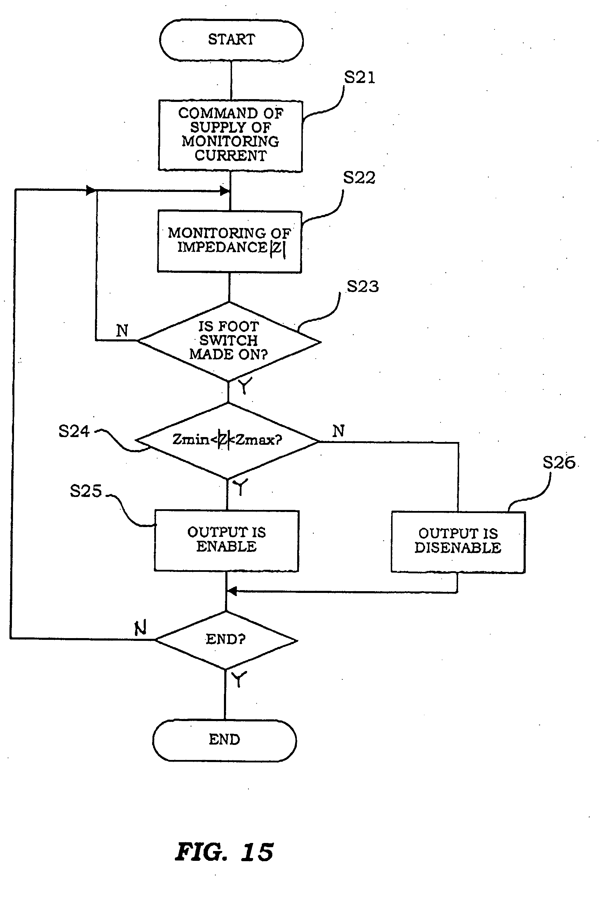

Upon ON-operation of the power switch 16 of the control drive unit 1, the computer 31 in the drive circuit 23 performs control (step S21 of Fig. 15) so that a weak monitoring current Im of a constant level is constantly flowed.

In the present embodiment, the magnitude of an output voltage from the output transformer 28 is in proportion to an input of an adder in the voltage control amplifier 25. This means that an output signal to the voltage control amplifier 25 indicates the magnitude of loading (i.e. impedance) for ultrasonic vibration.

In the present embodiment, a signal outputted from the differential amplifier 29, i.e. an input signal to the voltage control amplifier 25, is inputted to the computer 31 through the A/D converter 32. As a result, the computer 31 constantly monitors the magnitude of the impedance for the ultrasonic vibration (step S22 of Fig. 15).

An operator then carries out an ON-operation through the operation pedal 13 or the operation pedal 14 of the foot switch 3 (step S23 of Fig. 15) in an attempt to begin ultrasonic treatment under the output conditions (the output control patterns shown in the first embodiment or in their modifications) set through the setting device 21. In this case, the CPU 31A of the computer 31 exerts control, so that the ultrasonic output under the output conditions set through the setting device 21 is allowed to initiate only when the impedance | Z | being monitored is within a preset range (Zmin< | Z | <Zmax) (step S24), whereby the treatment of living tissues is effected by using the ultrasonic vibration (i.e., the output is enable: step S25). In contrast, when the impedance | Z | being monitored is outside the preset range (Zmin< | Z | <Zmax) (step S25), the treatment of living tissues is not effected by using the ultrasonic vibration (i.e., the output is disenable: step S26).

The threshold values Zmin and Zmax for the impedance are determined based on impedance values at the time when comparatively soft living tissues to be treated are held by the treatment portion 5 of the hand piece 2. Specifically, when no living tissues are being held by the treatment portion 5, the impedance is: | Z | <Zmin. In this state, no ultrasonic output is initiated under the output conditions set through the setting device 21 if an ON-operation is attempted through the operation pedal 13 or the operation pedal 14 of the foot switch 3. Further, when a metal hemostatic clamp or hard living tissues, such as bone is being held, the impedance is: Zmax< | Z |. In this state as well, no ultrasonic output is initiated under the output conditions set through the setting device 21 if an ON-operation is attempted through the operation pedal 13 or the operation pedal 14 of the foot switch 3.

In this way, the ultrasonic output is initiated only when the holding of the living tissues to be treated is ensured. Therefore, when treatment is effected under the output conditions where the magnitude of the ultrasonic waves is altered with the lapse of output time as shown in Fig. 5, for example, tissues can be reliably coagulated. In addition, since no ultrasonic output is initiated in the state of holding a metal hemostatic clamp or the like, the probe 9 can be prevented from being damaged.

It should be noted that the monitoring current Im may be permitted to flow for only Δt seconds (e.g., several hundreds milliseconds) after the ON-operation of the operation pedal 13 or the operation pedal 14 of the foot switch 3. In this case, the ultrasonic output is initiated after Δt seconds provided that the impedance | Z | I is: Zmin< | Z | <Zmax.

As described above, in the present embodiment, coagulation of living tissues can be reliably carried out as in the first embodiment. In addition, since damages on a probe can be prevented, good durability is ensured and repeated use is enabled, which, as a result, ensures realization of low costs.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An ultrasonic surgical apparatus comprising:
a treatment device with an ultrasonic transducer driven to generate ultrasonic vibration in response to supply of current;
an operation device outputting a signal commanding the ultrasonic transducer to start driving in response to an operator's operation;
a driver driving the ultrasonic transducer by supplying the current thereto; and
a controller controlling the driver to supply the current so that the current having a predetermined amount is supplied in response to the signal outputted from the operation device and then the current is reduced in amount as a time counted from starting the supply elapses.

2. The ultrasonic surgical apparatus according to claim 1,
wherein the controller includes means stopping the supply of the current automatically at a time when the time from starting the supply reaches a preset time.

3. The ultrasonic surgical apparatus according to claim 1,
wherein the controller is configured to control the driver based on an output control pattern with which the current of the predetermined amount is supplied in response to the signal outputted from the operation device and then the current is reduced in amount as the time counted from starting the supply elapses.

4. The ultrasonic surgical apparatus according to claim 3,
wherein the output control pattern is formed to stop the supply of the current automatically at a time when the time counted from starting the supply reaches a preset time.

5. The ultrasonic surgical apparatus according to claim 4,
wherein the output control pattern is preset to control energy to be given to the ultrasonic transducer in a period of time from the start of the current supply to the preset time so that an amount of energy is sufficient for only coagulation of an object's area to be treated with the ultrasonic transducer.

6. The ultrasonic surgical apparatus according to claim 3,
wherein the output control pattern is preset to stepwise decrease the amount of the current as the time counted from starting the supply elapses.

7. The ultrasonic surgical apparatus according to claim 3,
wherein the output control pattern is preset to gradually decrease the current from the predetermined amount to a further predetermined amount lower than the predetermined value, when a specified period of time elapses from starting the supply.

8. The ultrasonic surgical apparatus according to claim 3,
wherein the output control pattern is preset to change the current in pulsed waves from starting the supply and decrease at least one of an upper limit, a lower limit, and a duty ratio of the pulsed waves as the time counted from starting the supply elapses.

9. The ultrasonic surgical apparatus according to claim 3, comprising:
a detector detecting a signal indicative of voltage to be applied to the ultrasonic transducer;
means commanding a constant monitoring current to be supplied to the ultrasonic transducer;
means determining whether or not an amount of the voltage detected through the detector is within a predetermined range on condition that the monitoring current is in supply to the ultrasonic transducer; and
means making operations at the operation device effective, only when the amount of the voltage is within the predetermined range.

10. The ultrasonic surgical apparatus according to claim 3,
wherein the controller is configured to control the driver using selectively a first output control pattern and a second output control pattern, the first output control pattern being set to supply the current having a first current value under control of constant current control in response to the signal from the operation device and the second output control pattern being set to supply the current having a second current value in response to the signal outputted from the operation device and then the current is reduced in amount as the time counted from starting the supply elapses.

11. The ultrasonic surgical apparatus according to claim 10,
wherein the operation device outputs the signal in response to an operator's selective operation and is provided with a first switch and a second switch for selectively
commanding the ultrasonic transducer to be driven on the basis of the first and second output control patterns, respectively.

12. The ultrasonic surgical apparatus according to claim 11,
wherein the operation device is a foot switch.

13. The ultrasonic surgical apparatus according to claim 10,
wherein the second current value is higher than the first current value.

14. The ultrasonic surgical apparatus according to claim 10,
wherein at least one of the first and second output patterns are formed to stop the supply of the current automatically at a time when the time counted from starting the supply reaches a preset time.

15. The ultrasonic surgical apparatus according to claim 14,
wherein
the first output control pattern is preset to control energy to be given to the ultrasonic transducer in a period of time from the start of the current supply to the preset time so that an amount of energy is sufficient for coagulation and incision of an object's area to be treated with the ultrasonic transducer and
the second output control pattern is preset to control energy to be given to the ultrasonic transducer in a period of time from the start of the current supply to the preset time so that an amount of energy is sufficient for only coagulation of an object's area to be treated with the ultrasonic transducer.

16. The ultrasonic surgical apparatus according to claim 15,
wherein the second output control pattern is preset to stepwise decrease the amount of the current as the time counted from starting the supply elapses.

17. The ultrasonic surgical apparatus according to claim 15,
wherein the second output control pattern is preset to gradually decrease the current from the predetermined amount to a further predetermined amount lower than the predetermined value, when a specified period of time elapses from starting the supply.

18. The ultrasonic surgical apparatus according to claim 15,
wherein the second output control pattern is preset to change the current in pulsed waves from starting the supply and decrease at least one of an upper limit, a lower limit, and a duty ratio of the pulsed waves as the time counted from starting the supply elapses.

19. A program, of which data is stored in a memory and readable by a computer, applied to an ultrasonic surgical apparatus comprising a treatment device with an ultrasonic transducer driven to generate ultrasonic vibration in response to supply of current; an operation device outputting a signal commanding the ultrasonic transducer to start driving in response to an operator's operation; and a driver driving the ultrasonic transducer by supplying the current thereto, wherein
the program making the computer function as means for controlling the driver using selectively a first output control pattern and a second output control pattern, the first output control pattern being set to supply the current of a first current value under control of constant current control in response to the signal from the operation device and the second output control pattern being set to supply the current of a second current value in response to the signal outputted from the operation device and then the current is reduced in amount as the time counted from starting the supply elapses.

20. A control device for ultrasonic treatment, comprising:
an operation device allowing an operator to selectively command a first control mode and a second control mode, the first control mode allowing ultrasonic energy to be provided to a desired portion of a living body based on a first output control pattern so that a tissue of the desired portion is subjected to coagulation and then incision and the second control mode allowing ultrasonic energy to be provided to a desired portion of a living body based on a second output control pattern so that a tissue of the desired portion is subjected to coagulation; and
a controller controlling generation of the ultrasonic energy based on either the first output control pattern or the second output control pattern, in response to either the first control mode or the second control mode from the operation device.
